# Europäisches Patentamt

# European Patent Office

# Office européen des brevets

(19)

(11) Publication number: **0 162 907 B1**

# EUROPEAN PATENT SPECIFICATION

(12)

(45) Date of publication of patent specification: **15.01.92**

(51) Int. Cl.⁵: **C12Q 1/24**, C12M 1/00, C12M 1/34

(21) Application number: **85900274.3**

(22) Date of filing: **08.11.84**

(86) International application number:
**PCT/US84/01829**

(87) International publication number:
**WO 85/02201 (23.05.85 85/12)**

(54) **SYSTEM AND METHODS FOR CELL SELECTION.**

(30) Priority: **08.11.83 US 550233**

(43) Date of publication of application:
**04.12.85 Bulletin 85/49**

(45) Publication of the grant of the patent:
**15.01.92 Bulletin 92/03**

(84) Designated Contracting States:
**AT BE CH DE FR GB LI LU NL SE**

(56) References cited:
**EP-A- 0 094 193**
**EP-A- 0 132 064**
**US-A- 4 055 799**
**US-A- 4 326 934**

**LABORATORY EQUIPMENT DIGEST, vol. 18,
no. 10, October 1980, pages 91-93, US;
"High-voltage FFE separates cells without
damage"**

**See also documents of WO85/02201**

(73) Proprietor: **SCIENTIFIC DIAGNOSTICS, INC.
375 Park Avenue Suite 2201
New York, NY 10152(US)**

(72) Inventor: **WEINREB, Arye
8 Shiler Street
Jerusalem(IL)**
Inventor: **DEUTSCH, Mordechai
14 Don Yosef Nassi St.
Petah-Tikvah(IL)**

(74) Representative: **Miller, Joseph et al
J. MILLER & CO. Lincoln House 296-302 High
Holborn
London WC1V 7JH(GB)**

EP 0 162 907 B1

## Description

The present invention relates to a method and apparatus for placing individual living cells at identifiable locations. More generally, the invention also concerns equipment and methods for studying, examining, or manipulating large groups of living cells, e.g. 10,000 or more individual cells, on a cell-by-cell basis.

In Swiss Patent Application Serial No. 2897/82-3, filed May 10, 1982 we described equipment and methods for studying living cells on a cell-by-cell basis.

Briefly, our Swiss application described a process for placing individual living cells at identifiable locations comprising the steps of:

(a) providing a carrier having a plurality of apertures, the apertures being arranged in an ordered array and being sized to hold individual cells;

(b) applying a fluid containing living cells to the carrier; and

(c) applying a force to the cells to move the cells into the apertures.

Once in their individual apertures, the cells are studied, examined, and manipulated on a one-by-one basis. For example, the cells in the carrier apertures can be subjected to biological tests and particular properties of individual cells can be measured. As described in detail in our Swiss application, a particularly important application of this analysis approach involves using it to perform the Cercek SCM (Structuredness of Cytoplastic Matrix) test for diagnosing cancer. See for example, L. Cercek et al, Biophys. J., July 1978, Vol. 23, No. 1, p.395 ff.

The present invention relates to new equipment and methods for practicing and using the methods and equipment described in our prior Swiss patent application.

According to one aspect of the present invention, there is provided a method for placing individual living cells at identifiable locations comprising the steps of:

(a) providing a substantially planar carrier made of a material selected from group consisting of metal with a superimposed inorganic coating, plastic with a superimposed inorganic coating, uncoated metal and uncoated plastic, said carrier having a plurality of apertures which are arranged in an ordered two-dimensional array and are sized to hold individual cells;

(b) applying a fluid containing living cells to the carrier;

(c) applying an electromagnetic field to the cells to drive the cells into the apertures; and

(d) washing the carrier to remove excess cells and debris.

According to another aspect of the present invention, there is provided apparatus for placing individual cells at identifiable locations comprising:

(a) a substantially planar carrier made of a material selected from group consisting of metal with a superimposed inorganic coating, plastic with a superimposed inorganic coating, uncoated metal and uncoated plastic, the carrier having a plurality of apertures which are arranged in an ordered two-dimensional array and are sized to hold individual cells;

(b) means for applying an electromagnetic force to the cells to drive the cells into the apertures.

In its preferred form, the invention includes methods and apparatus for loading cells onto a carrier and preventing them from leaving a carrier; improved methods and equipment for exchanging the bathing fluid surrounding cells captured in the apertures of a carrier; improved methods and equipment for selecting and removing particular cells from a carrier; improved methods and equipment for washing excess cells from a carrier; and improved carriers and methods for constructing such carriers.

In certain embodiments of the invention, crossed electric and magnetic fields are used for loading, while in other embodiments, an electric field normal to the surface of the carrier is used.

In accordance with other embodiments of the invention, a time varying magnetic field is used to enhance the rate at which bathing fluid surrounding cells captured in the apertures of a carrier is exchanged. In addition to enhancing the rate of fluid exchange, such a time varying magnetic field also has a massaging effect on the cells captured in the apertures.

In accordance with additional embodiments of the invention, cells are held in the apertures of a carrier by adjusting the osmolarity of the bathing solution surrounding the cells so as to cause the cells to swell.

In accordance with further embodiments of the invention, electromagnetic fields are used to select and remove particular cells from a cell carrier. In certain embodiments of the invention, a time varying electric field and a constant magnetic field is used to select and remove cells having a particular charge to mass ratio. In other embodiments, a charged probe is used to remove individual cells from the carrier.

In accordance with additional embodiments of the invention, equipment and methods are provided for washing excess cells from the surface of a cell carrier wherein a pressure differential is applied across the cell carrier during the washing process. In certain preferred embodiments of the invention, the carrier is washed by supplying fluid to its top surface through an inflow tube and removing it through a drain tube.

In accordance with still further embodiments of the invention, improved cell carriers and methods for producing such carriers are provided. In particular, the carrier may have apertures which include at least one vertical wall. The latter carriers are conveniently prepared using an ion bombardment process.

Further objects and features of the present invention will become more fully apparent from the following description of several embodiments of the invention based on the accompanying drawings, wherein:-

Figures 1A-1E are schematic illustrations, partly in sectional view, of preferred cell carriers which may be used in the invention;

Figures 2-4 are scanning electron micrographs of copper carriers for use with the present invention;

Figure 5 is a scanning electron micrograph showing a copper carrier coated with silicon;

Figure 6 is a scanning electron micrograph showing a copper carrier having square-shaped apertures which are sized to hold and retain lymphocytes having a cross-sectional size of approximately 7$\mu$m;

Figure 7 shows a typical experimental arrangement suitable for loading cells into carriers of the types shown in Figures 2-4;

Figure 8 is a scanning electron micrograph showing a carrier filled with lymphocytes;

Figures 9-10 are scanning electron micrographs showing individual cells in individual apertures of a carrier;

Figure 11 is a scanning electron micrograph showing the surface of the carrier prior to washing;

Figure 12 shows the use of an electric field to drive cells into the apertures of a carrier;

Figure 13 shows the use of crossed electric and magnetic fields to drive cells into the apertures of a carrier;

Figures 14-15 show the use of a time varying magnetic field to enhance fluid exchange about cells captured in a carrier;

Figure 16 shows the use of a time varying E field crossed with a constant B field to select a sub-population of cells captured in a carrier based on their charge to mass ratio.

In the preferred form of the invention described below, the present invention relates to improved equipment and methods for studying, examining or manipulating living cells on a one-by-one basis wherein individual living cells are placed at identifiable locations on a cell carrier.

The cell carrier has an array of cell receiving holes, where for each hole, the location in the array, or address, is fixed and known. The holes extend from the carrier top side to a spaced apart bottom side. The holes have preselected configurations so that when a batch of cells passes over the carrier top side only the carrier top side only preselected cells, based on their particular size, enter and become supported in the holes. Cells of sizes smaller than those of the selected cells pass through the holes, while much larger cells cannot enter the holes. Once the carrier is rinsed, only selected cells are located in its holes, one cell per hole at a fixed address.

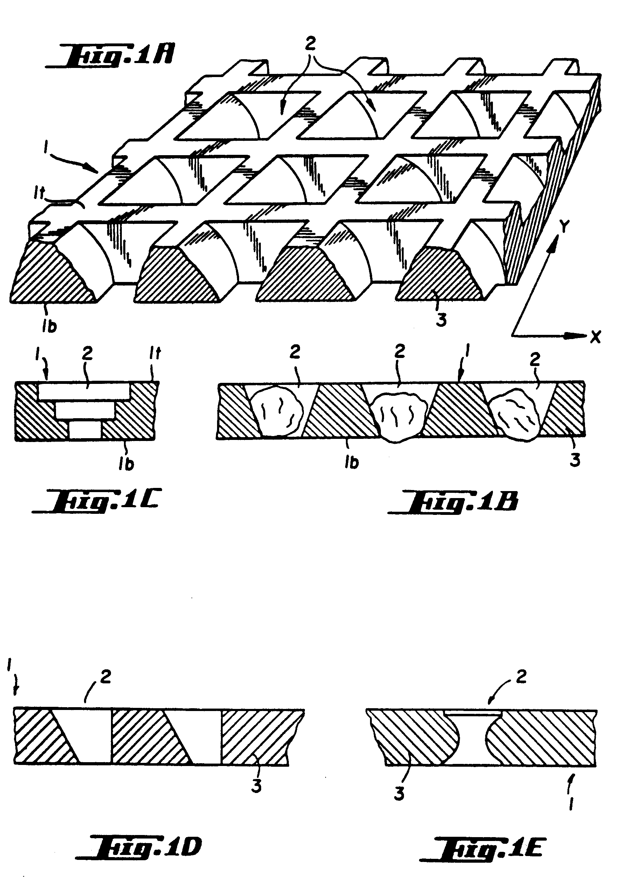

Various cell carrier configurations are shown in Figures 1A-1E. Carrier 1 includes base 3 in which are formed apertures or holes 2. The apertures or holes, as well as their arrangement, may have various configurations. In Figure 1A, the holes are arranged in rows and columns along axes X and Y, respectively. As shown in Figure 1B, the holes have larger openings at their tops than at their bottoms. The side walls of the apertures may converge continuously towards the opening at the bottom side 1b of the cell carrier, or in steps, as shown in Figure 1C. Also, as shown in Figure 1D, not all sides of the aperture need slope inwardly. Rather, a portion of the walls of the aperture can be essentially vertical so as to help capture and retain the cells in the apertures, especially when the cells are introduced into the carrier by being flowed across the top of the carrier in a direction substantially perpendicular to the apertures' vertical walls.

The shape of apertures 2 enables the cells to be effectively held to the carrier by applying means, such as a pressure difference between the upper and the bottom side of the carrier, or electromagnetic forces. Briefly, to first separate a particular group of cells from cells of other groups, since the cells in each group are of known size or sizes, which typically differ from those in other groups, the carrier 1 is chosen to have holes of sizes so that when the matter, e.g., blood, containing the various cell groups is placed on the carrier 1, effectively most if not all of the holes are occupied by cells of the group of interest, one cell per hole.

For example, with regard to the SCM test referred to above, the holes are sized to be suited for receiving lymphocytes, among which there are two main sizes of about 7 um and about 10-15 um, the 7 um lymphocytes being the cells of interest. To capture and retain this population of cells, it has been found that at the upper surface or side 1t of carrier 1, the apertures should have a cross-sectional dimension of approximately 10 um and that at the bottom surface or side 1b, they should have a cross-sectional dimension of approximately 5 um. In this way, the desired population of cells can easily enter the aperture without suffering substantial damage and yet, once in the aperture, the cells cannot pass out of the bottom of the carrier.

In general the aperture should be shaped so that either at its bottom side or at a cross-section intermediate sides 1t and 1b, the cross-sectional dimension is less than at the top side, so that a desired cell entering an aperture does not pass through the aperture, but rather is held therein. Fig. 1E illustrates an aperture configuration wherein the minimum cross-section is located in a plane intermediate between the top and bottom sides of the carrier. In addition to properly selecting the aperture's entering and exiting dimensions, it is also important to choose the carrier thickness between the top and the level of the minimum cross-sectional dimension so that the size of the aperture is related to the size of the desired cells so that when a desired cell enters an aperture practically the entire cell is within the aperture, thus preventing it from being washed out during washing of the carrier.

The carrier 1 is made of metal such as copper, gold, nickel, silver or others, or of plastic.

In addition to using pure metal or plastic carriers, in some cases it is desirable to coat the carrier with various materials in order to change either or both of its chemical and mechanical surface characteristics. Examples of suitable coating materials include silicon, silicon dioxide and various inorganic glasses. When using such coating materials, or for that matter, when choosing a material from which to make an uncoated carrier, it is important to determine that the material does not interact with the cells in a way which will interfere with the test or tests to be performed.

For example, with regard to the SCM test referred to above, it has been found that a coating of $SiO_2$ on the carrier leads to activation of the cells (lymphocytes) which masks the response of these cells to stimulating agents. A similar activation is found with a mixture of silicon and $Si_2O_3$. Pure silicon, on the other hand, does not lead to activation of the cells. Accordingly, for the SCM test, a carrier coating of silicon is appropriate, while a coating of silicon dioxide or silicon plus $Si_2O3$ is not. Similar selections of coating materials can be readily made by persons skilled in the art for other types of diagnostic tests.

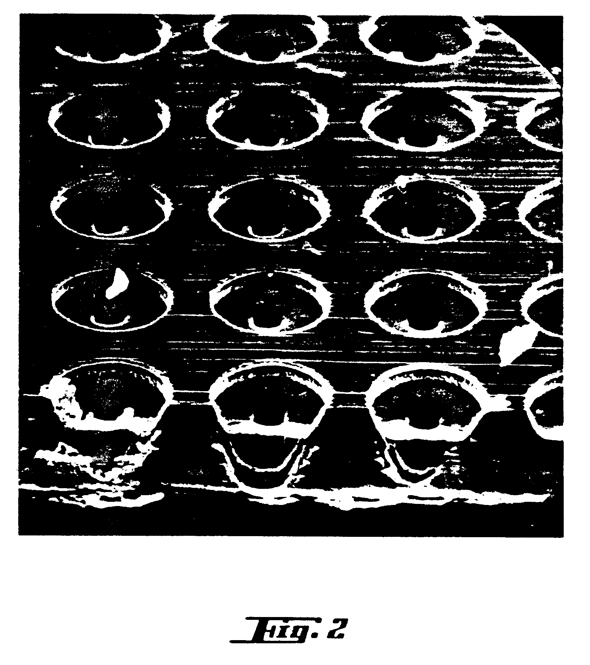

Scanning electron micrographs of a copper carrier for use with the present invention are shown in Figs. 2-4. Fig. 2 shows the top surface of the carrier at a magnification of 1000 X. At the level of this surface, the apertures have a cross-sectional dimension (diameter) of approximately 11 microns. The minimum cross-sectional dimension for these apertures is located in a plane intermediate the carrier's top and bottom surfaces and has a magnitude of approximately 4 microns. The spacing between this intermediate plane and the top surface of the carrier is approximately 6 microns. The spacing between apertures is approximately 15 microns. In general, the inter-aperture spacing should be kept as small as possible so as to maximize the chances that cells will come to rest inside apertures rather than on the portions of the carrier between apertures.

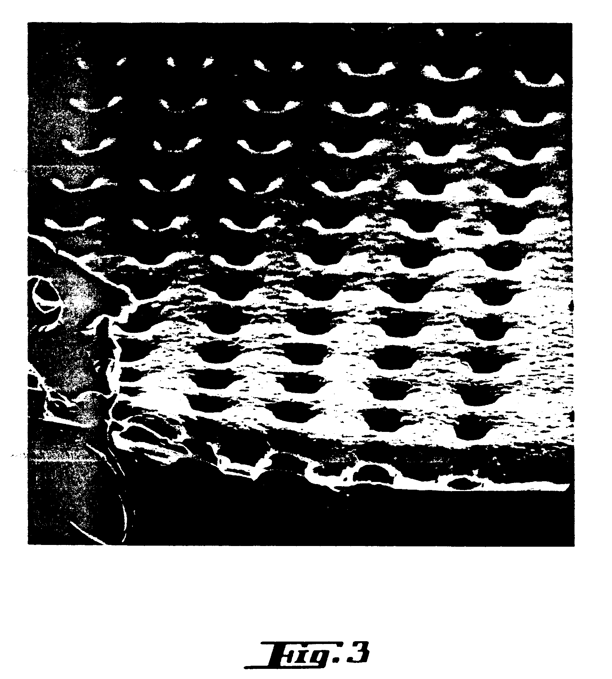

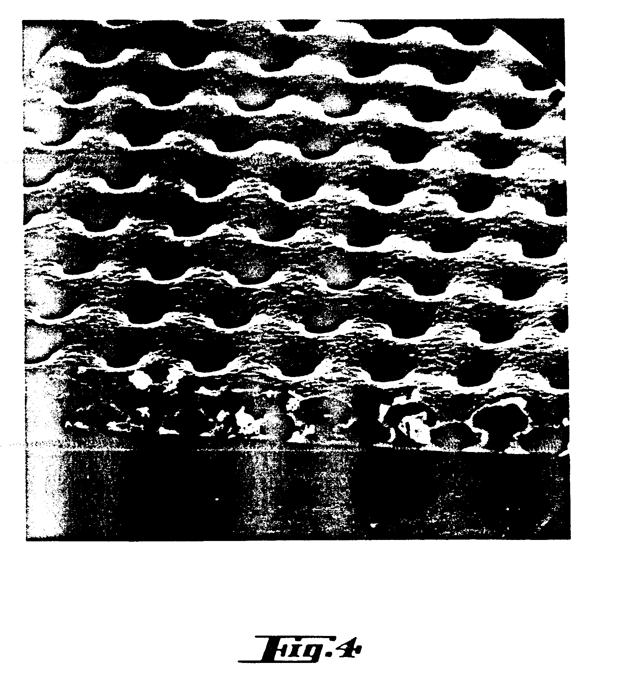

Figs. 3 and 4 show the bottom surface of the carrier of Fig. 2 at a magnification of 1000 X. Fig. 3 also shows a turned-up corner of the carrier. Examining the edges of the carriers of Fig. 2-4 reveals that the apertures have a vertical cross-sectional configuration of the type shown in Fig. 1E.

The carrier shown in Figs. 2-4 was prepared using a standard photo-etching technique of the type commercially employed to make transmission electron microscope grids. As is known in the art, that process, in its last stages, involves the deposition of metal on one side of a preformed grid so as to increase the strength of the grid. When a transmission electron microscope grid is to be formed, the deposition step is carried on only for a short time so as to keep the size of the apertures as large as possible, i.e., to minimize the width of grid members which in turn minimizes the interference of the grid with the transmission of electrons through the specimen and to the electron detector. To form the carrier of Figs. 2-4, the deposition step, rather than being short, was continued for a relatively long period of time until enough metal was deposited on the back surface of the grid to fill in the apertures to the extent shown in the figures. As shown most clearly in Fig. 4, the deposited metal (copper) built up on the solid parts of the grid and overlapped into the apertures to close off the apertures and thus form the desired minimum cross-sectional dimension of the apertures.

Rather than using a deposition process to form carrier apertures of the desired configuration, other processes, in particular ion bombardment processes through masks of different thicknesses and the like, can be used. Such processes are particularly useful in preparing asymmetric apertures, such as the apertures shown in Fig. 1D.

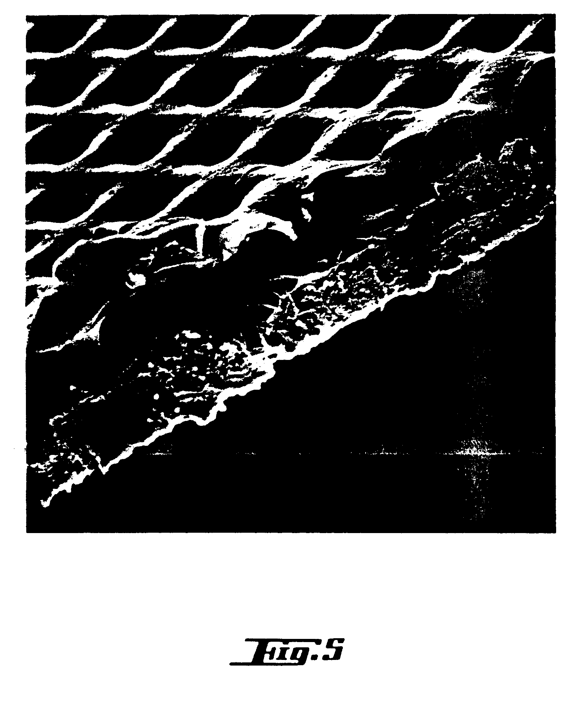

Fig. 5 is a scanning electron micrograph at a magnification of 720 X illustrating a coated carrier. The base carrier in this case was formed from copper and the coating is pure silicon which was deposited on the carrier by vapor deposition. As can be seen in Fig. 5, coatings can be used to change (reduce) the cross-sectional dimensions of the apertures, as well as to provide an especially smooth and/or inert surface for contacting the cells.

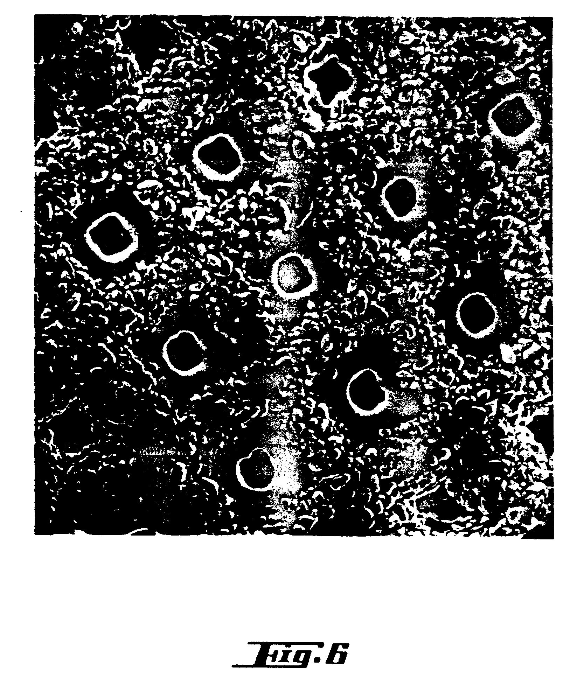

Fig. 6 shows another uncoated, copper carrier, in this case having square rather than circular apertures. The cross-sectional dimension of the apertures at the top surface of this carrier is approximately 10 microns and the minimum cross-sectional dimension is approximately 5 microns. The minimum cross-sectional dimension lies in a plane approximately 7-8 microns below the top surface of the carrier. The spacing between apertures is approximately 12 microns.

The carriers of Figs. 2-4 and 6 are sized to be particularly well suited to capturing and retaining lymphocytes having a cross-sectional size of approximately 7 um. As will be evident to persons skilled in the art from the disclosure herein, other carriers having different aperture configurations can be constructed for capturing and retaining cells of different types and sizes.

As previously pointed out, the holes 2 in carrier 1 are regularly arranged over or in the carrier, e.g., in rows and columns, to enable a clear identification of the position of very hole 2, for example, by its X and Y coordinates in the plane of the carrier. In the described embodiment the holes are disposed in rows and columns, extending perpendicularly to each other, thereby forming a matrix-like structure. The number of holes is chosen depending on the number of cells to be carried. For example, with 100 holes per row and column there is a total of 10,000 holes to carry 10,000 cells on the carrier of the described embodiment, each with its unique position in X and Y.

To practice the method of the present invention, a few drops of the solution containing the cells, e.g., blood containing the lymphocytes, are dripped onto the cell carrier. An electromagnetic field, together, if desired, with another force, for example, a pressure differential, is applied across the carrier to move the cells into the apertures. The liquid passes through the holes in the carrier. However, the cells remain on the carrier. Since the sizes of the holes 2 are chosen to accommodate lymphocytes only, they enter the holes. Each hole accommodates only one cell. Excessive and other cells may be washed off the surface of the carrier, such as cells of sizes so great that they can't enter any hole, and/or excess cells more than the number of holes. Thereafter, in order to prevent the cells in the holes from leaving the carrier, they may be fixed thereto by various means, e.g., by applying a continuous pressure differential across the holes, by changing the osmolarity of the bathing solution to cause the cells to swell, by covering the carrier by an adhesive, colloidable matter, and by electrically charging the carrier, as well as by external electric and/or magnetic fields.

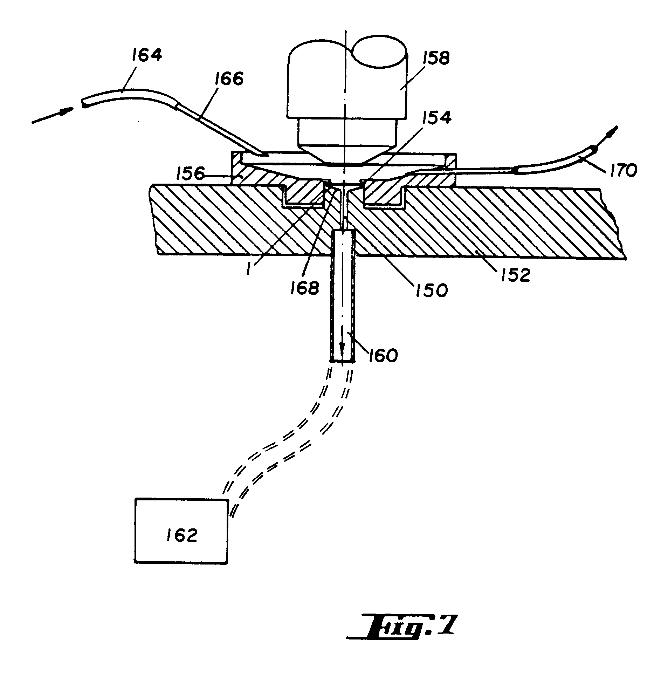

Fig. 7 illustrates a typical experimental arrangement which has been used to load cells into carriers of the types shown in Figs. 2-6.

Carrier 1 is held in place above orifice 150 in plate 152 by means of collar 154 of solution basin 156. The collar presses the carrier against the portion of plate 152 which surrounds orifice 150 and creates a seal between that portion and the carrier. This seal prevents substantial numbers of cells from passing around the edges of the carrier, rather than being captured in the apertures.

Orifice 150 is connected by outflow tube 160 to pump 162. The pump serves to produce a pressure differential across carrier 1 which pulls the cells into the apertures in the carrier. It has been found that a more uniform filling of carrier 1 can be achieved by providing a shallow taper 168 at the mouth of orifice 150. This taper reduces the amount of time required to fill the apertures at the perimeter of the carrier.

Basin 156 is configured so as to allow microscope objective 158 to be brought close enough to carrier 1 so that the apertures in the carrier can be brought into focus. Solutions are provided to basin 156 by one or more inflow tubes 164 which are conveniently connected to syringe needles 166. The inflow tubes are used to introduce various bathing and reagent solutions to basin 156. The inflow tubes are also used to wash excess cells off the top surface of carrier 1. In this case, fluid is removed from basin 156 by means of drain tube 170. Cells are applied to carrier 1 using a standard syringe. During this operation microscope objective 158 and basin 156 are moved apart to allow ready access to carrier 1. The level of fluid in basin 156 is monitored during the testing of cells and, as necessary, fluid is added to the basin to keep the cells captured in carrier 1 continuously submerged in liquid.

A typical procedure used to capture and retain human lymphocytes in a carrier using the apparatus of Fig. 7 was as follows. First, a sample of human whole body was obtained in the standard way. A plasma fraction of this blood was then obtained by either centrifuging the sample at approximately 100 g for approximately 6 minutes or by incubating the sample at 37°C for approximately a half an hour. In either case, the plasma fraction had a pinkish cast indicating the presence of red blood cells. The red blood cell/white blood cell ratio of the plasma fractions used to fill carriers was estimated to be approximately 30/1.

Once obtained, the plasma fractions were diluted with phosphate buffered saline until a cell concentration of either approximately $6 \times 10^6$ cells/cc or $12 \times 10^6$ cells/cc was reached. So that the cells would fluoresce and thus be easily seen under the microscope during loading onto the carrier, fluorescein diacetate (FDA) was added to the cell suspension at a concentration of approximately 2.5 uM and the cells were incubated with FDA for approximately 10-30 seconds prior to being applied to the carrier.

To make sure that the carrier was free from contamination, phosphate buffered saline was added to basin 154 with carrier 1 in place and pumped through carrier 1 by means of pump 162. Pump 162 was adjusted to produce a pressure differential across carrier 1 in the range of 0.5-5.0 cm of water.

The cells were applied to carrier 1 by bringing a standard syringe containing the cell suspension into the vicinity of the carrier. For the $6 \times 10^6$ cells/cc concentration it was found that three drops of the cell suspension applied near to, but not directly on, the carrier were adequate to essentially fill all of the apertures in a carrier having approximately 7500 holes, while for the $12 \times 10^6$ cells/cc concentration level and the same size carrier, one drop applied directly to the carrier was found to be sufficient. In either case, essentially complete filling of the carrier occurred within a period of seconds to minutes, depending on how well collar 154 sealed the carrier to plate 152.

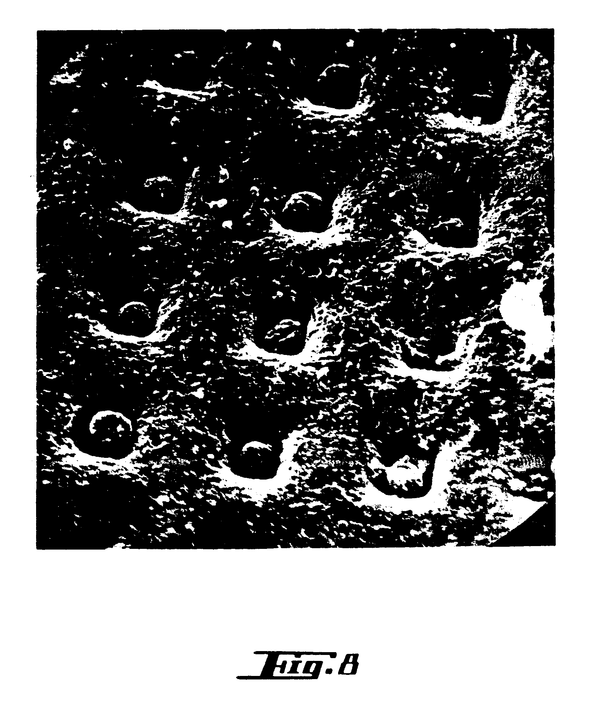

Fig. 8 is a scanning electron micrograph at a magnifications of 1000 X showing the carrier filled with lymphocytes. The fixation process used to prepare this micrograph causes the cells to contract. This makes them appear somewhat smaller than the apertures. When the cells were alive, they essentially filled the whole aperture with their tops at or just below the top surface of the carrier.

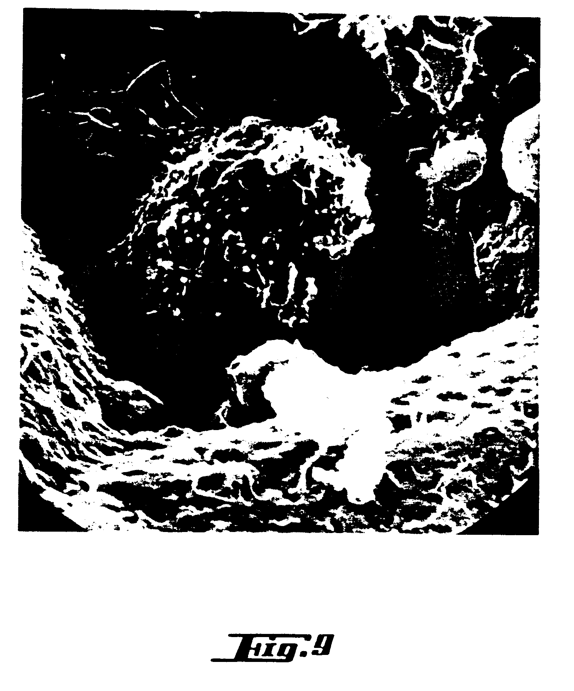

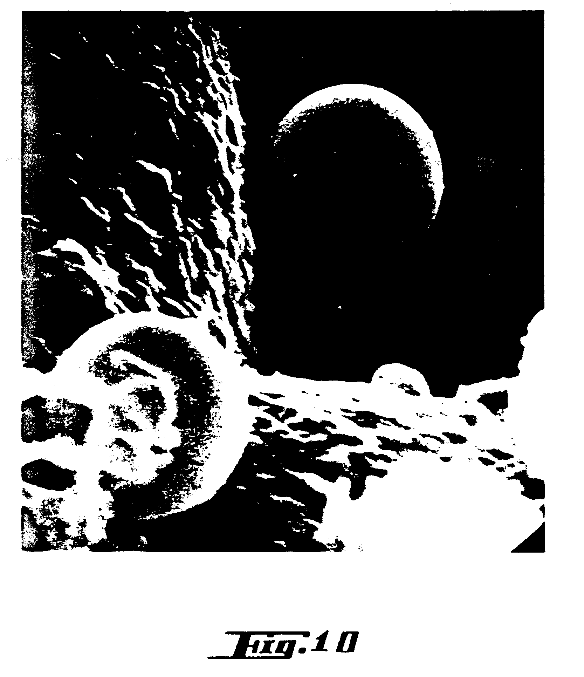

Figs. 9-10 are scanning electron micrographs at a magnification of 6600X showing individual cells in individual apertures. The cell shown in Figs. 9 is a lymphocyte, while the cell in the aperture in Fig. 10 is an erythrocyte. Because erythrocytes are smaller than lymphocytes and are relatively flexible, if pressure had continued to be applied across the carrier, the erythrocyte shown in Fig. 10 would have passed down and out of the aperture.

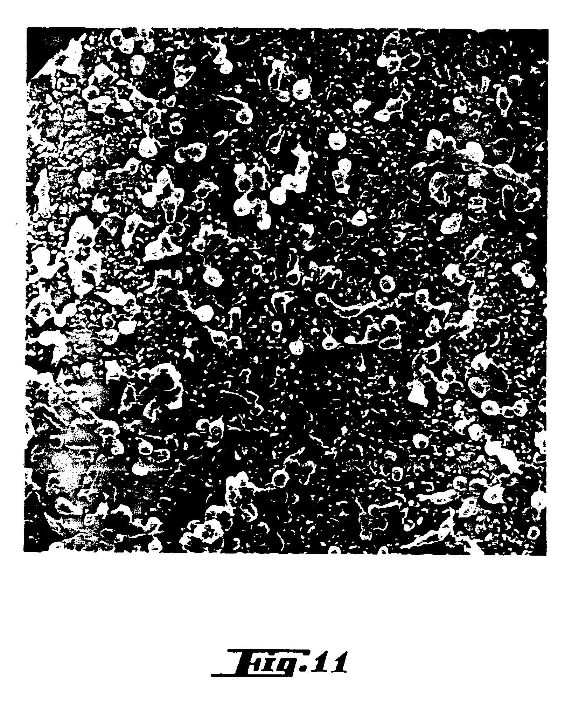

Fig. 11 is a scanning electron micrograph at a magnification of 480X showing the carrier surface prior to washing to remove excess cells and debris. For the apparatus of Fig. 7, washing is done using inflow tube 164 and drain tube 170. Note that the pressure differential created by pump 162, as well as the configuration of the apertures, serves to hold the cells in their apertures during the washing process.

As can be seen in Fig. 11, prior to washing there are individual lymphocytes in individual apertures, but the top of the carrier is covered with both excess lymphocytes and erythrocytes, as well as other cell types and debris. Comparing Fig. 11 with Fig. 8, which shows the carrier surface after washing, clearly demonstrates the effectiveness of the washing process in removing excess cells and debris.

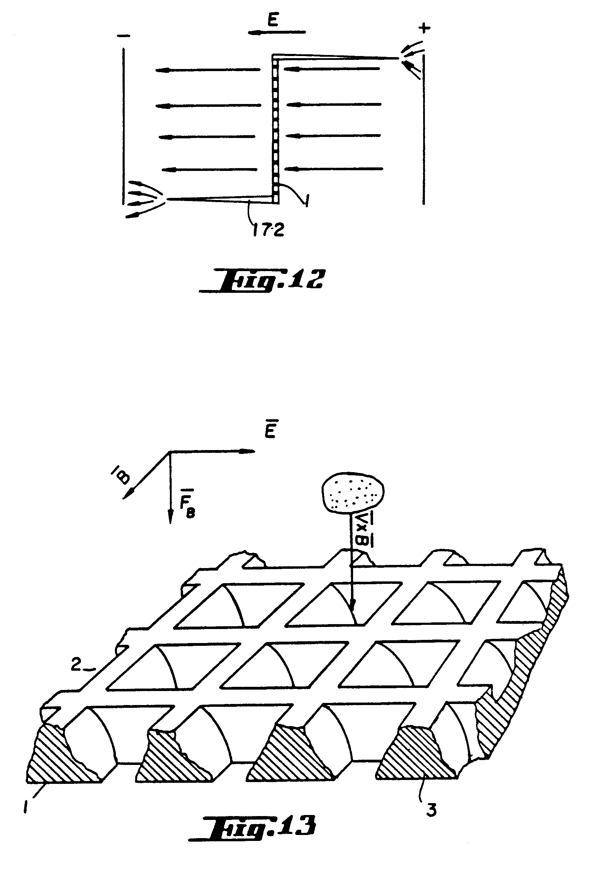

For example, Fig. 12 shows the use of an electric field to drive the cells against the carrier and into the apertures. The field is oriented perpendicular to the top surface of the carrier. As is known in the art, biological cells, including lymphocytes, normally carry a net electrical charge, or, by adjusting the pH or other parameters, can be made to have a net charge. The electric field shown in Fig. 12 will accordingly cause cells, e.g., positively charged cells, to move towards the carrier and into the apertures, as desired. Of course, if it is negatively charged cells which one wants to capture on the carrier, one only needs to reverse the direction of the electric field.

The use of an electric field as the driving force can lead to electrolysis problems with uncoated metallic carriers. One solution to this problem is to coat the carrier with a non-conductor, as described above. Another solution, illustrated in Fig. 12, is to give the carrier a shape which localizes most of the electrolysis effects at points distant from the apertures where the cells are captured. In particular, in Fig. 12, the carrier is provided with ears or projections 172 which concentrate the electric field and thus the ionic current and electrolysis effects in regions away from the main body of the carrier. Such ears will also attract cells, but in general there will be an abundant excess of cells so that even if there is some concentration of cells in the regions of the ears, there will still be enough cells near the body of the carrier to fill the apertures.

As an alternative to using an electric field oriented perpendicular to the surface of the carrier, crossed electric and magnetic fields parallel to the surface of the carrier can be used to drive the cells into the apertures. As shown in Fig. 13, in this case, the electric field causes the charged cells to move across the top of the carrier, while the magnetic field produces a $v \times B$ force towards the surface of the carrier for positively charged cells. Again, negatively charged cells can be selected by reversing the direction of the B field. The use of a B field to drive the cells into the apertures has the advantage that once the cell comes to rest in the aperture, the force on the cell due to the driving force ceases because v is now equal to zero. In contrast, a pressure differential driving force continues to exert a force on the cells even after they have been captured in apertures, although in general this force is too small to cause damage to the cells.

In addition to using E and B fields to apply cells to the carrier, these fields can be used to enhance the rate of fluid exchange around individual cells and to select specific cells captured on the carrier based on such parameters as the cell's charge to mass ratio.

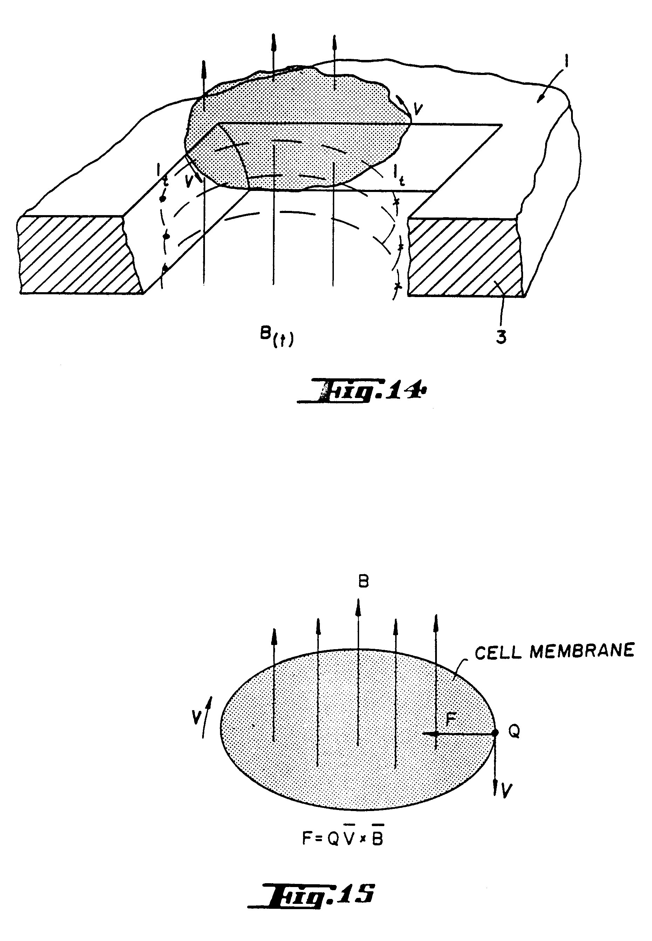

With regard to fluid exchange, Fig. 14 shows the use of a time varying magnetic field normal to the surface of the carrier to cause cells to rotate about their axes inside apertures. More specifically, the time varying magnet field generates a circular or tangential electric field parallel to the plane of the carrier. The magnitude and direction of such a field is described by Maxwell-Faraday law, also known as Lenz's law. This field acts on the fixed charges on the surface of the cell membrane and thus causes the cells to rotate about an axis parallel to the magnetic field. It should be noted that once the cells begin to rotate their cell membranes will experience either an inward or outward squeezing force resulting from the v x B (Lorentz) interaction between the charges on the membrane and the applied B field (see Fig. 15). Whether the force is inward or outward will depend on the sign of the cell's surface charge and the orientation of the B field. In essence, the time varying magnetic field, in addition to rotating the cells, will also have a massaging effect on them. Furthermore, there will be a tendency for the rotating cell, which in effect is a magnetic dipole, to move parallel to the magnetic field. In addition to the effects on the cells, the field also interacts with the charged ions in the bathing medium causing them to move in circular paths.

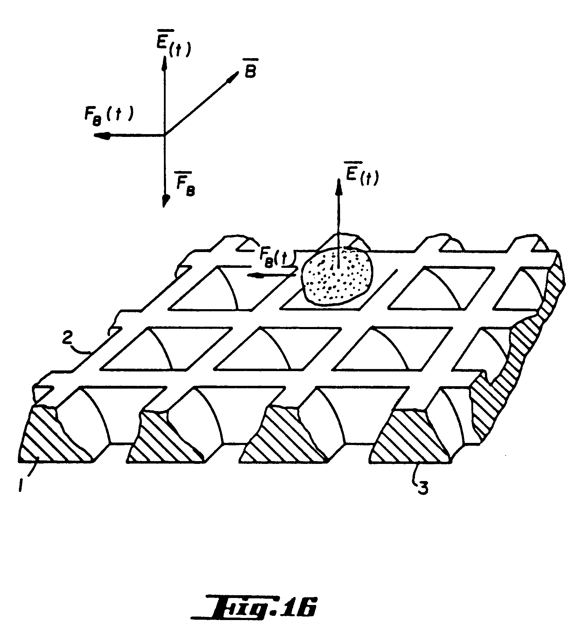

With regard to selecting particular types of cells from among the population captured on the carrier, Fig. 16 shows an arrangement for selecting those cells having a particular charge to mass ratio. As shown in that figure, a time varying, e.g., sinusoidal, electric field is applied across the carrier and a constant magnetic field is applied parallel to the top surface of the carrier. The response of individual cells to the electric field will depend on the frequency of the field and the cell's charge to mass ratio. Accordingly, by varying the frequency of the electric field, specific subgroups of cells can be made to move sufficiently far out of their apertures so that the force due to the magnetic field acting on the moving cell will cause it to move in the plane of the surface of the carrier. By means of surface washing during this process, these selected cells can be removed.

In addition to the foregoing, electric fields can be used to select individual cells. For example, individual cells can be removed from the carrier by a local electric field created by bringing a charged probe into the vicinity of a particular cell's aperture. Groups of cells can be similarly removed from the carrier and moved to a desired location by using a movable array of probes, where selected probes in the array can be charged to a value sufficient to attract and move a cell from its aperture.

**Claims**

1. A method for placing individual living cells at identifiable locations comprising the steps of:
   (a) providing a substantially planar carrier made of a material selected from group consisting of metal with a superimposed inorganic coating, plastic with a superimposed inorganic coating, uncoated metal and uncoated plastic, said carrier having a plurality of apertures which are arranged in an ordered two-dimensional array and are sized to hold individual cells;
   (b) applying a fluid containing living cells to the carrier;
   (c) applying an electromagnetic field to the cells to drive the cells into the apertures; and
   (d) washing the carrier to remove excess cells and debris.

2. A method according to claim 1, wherein the cells are held in the apertures of the carrier by effecting a subsequent step of adjusting the osmolarity of the bathing solution surrounding the cells so as to cause the cells to swell.

3. A method according to either claim 1 or claim 2, wherein the electromagnetic force is produced by an electric field oriented perpendicular to the surface of the carrier, or by crossed electric and magnetic fields oriented parallel to the surface of the carrier.

4. A method according to any of the preceding claims, wherein the bathing fluid surrounding cells captured in the apertures of the carrier is changed, while applying a time varying magnetic field normal to the surface of the carrier to cause the cells to rotate about their axes inside the apertures.

5. A method according to any of the preceding claims, which comprises the further steps of applying a time varying electric field across the carrier and applying a constant magnetic field parallel to the surface of the carrier, in order to move cells having a particular charge to mass ratio out of their apertures, whereby such cells are separated from cells remaining in the carrier apertures, and optionally washing the surface of the carrier while said electric and magnetic fields are being applied.

6. A method according to any of claims 1 to 4, wherein there is removed at least one individual cell from its aperture, by further steps comprising the steps of charging at least one probe and subjecting the at least one cell to the local electric field produced by the at least one charged probe to move the at least one cell out of its aperture.

7. A method according to claim 6, wherein there is charged array of probes and a plurality of cells is subjected to the local electric fields produced by the array to move the cells out of their respective apertures.

8. A method according to any of the preceding claims wherein step (d) is effected while applying a pressure differential across the carrier to hold the cells in the apertures.

9. A method according to any of the preceding claims, wherein step (d) is effected by supplying fluid to the top surface of the carrier through an inflow tube and removing it through a drain tube.

10. Apparatus for placing individual cells at identifiable locations comprising:

(a) a substantially planar carrier made of a material selected from group consisting of metal with a superimposed inorganic coating, plastic with a superimposed inorganic coating, uncoated metal and uncoated plastic, the carrier having a plurality of apertures which are arranged in an ordered two-dimensional array and are sized to hold individual cells;

(b) means for applying an electromagnetic force to the cells to drive the cells into the apertures.

11. Apparatus according to claim 10, wherein the means for applying an electromagnetic force includes means for producing at least one of the following, namely:

an electric field oriented perpendicular to the surface of the carrier; and

crossed electric and magnetic fields oriented parallel to the top surface of the carrier.

12. Apparatus according to either claim 10 or claim 11, wherein said carrier is made of a material selected from group consisting of metal with a superimposed inorganic coating, and plastic with a superimposed inorganic coating, said metal and plastic being conductors and said inorganic coating being a nonconductor.

13. Apparatus according to any of claims 10 to 12, wherein the carrier is made of metal and has a configuration which localizes electrolysis effects at points distant from the locations of the apertures.

14. Apparatus according to claim 13, wherein the carrier includes projections which extend away from the portion of the carrier which includes the apertures.

15. Apparatus according to any of claims 10 to 14, which includes additionally means for supplying cell bathing fluid to the carrier and for withdrawing it from the carrier.

16. Apparatus according to claim 15, which includes also means for producing a time varying magnetic field normal to the surface of the carrier to cause the cells to rotate about their axes inside the apertures.

17. Apparatus according to any of claims 10 to 16, which further includes electromagnetic means for ejecting cells which have a particular charge to mass ratio from among a population of cells captured in the apertures of a carrier.

18. Apparatus according to claim 17, wherein said electromagnetic means for ejecting cells comprises first and second means for respectively producing electric and magnetic fields, said electric field being time varying and being directed across the carrier, said magnetic field being constant and being directed parallel to the surface of the carrier, said electric field being adapted to cause cells which have the particular charge to mass ratio to move sufficiently far out of their apertures so that said magnetic field can move the cells in a plane parallel to the surface of the carrier.

19. Apparatus according to either claim 17 or claim 18, which further comprises means for washing the surface of the carrier.

20. Apparatus according to any of claims 10 to 19, which includes also means for removing at least one cell contained in an aperture of the carrier comprising at least one probe and means for charging the at least one probe to a value sufficient to attract and move the at least one cell from the aperture(s).

21. Apparatus according to claim 20, wherein said at least one probe comprises an array of probes and means for charging at least selected probes in the array to a value sufficient to attract and move a plurality of cells from their respective apertures.

22. Apparatus according to any of claims 10 to 21, wherein said carrier defines first and second outer surfaces and comprises an ordered two-dimensional array of apertures therethrough, the positions on the carrier of the apertures being identifiable and the apertures being sized to contain individual living cells therewithin in that the apertures have (i) a first cross section at the first outer surface of the carrier of such dimensions that living cells can pass through the first cross section without suffering substantial damage, (ii) a second cross section at a level spaced from the first outer surface of such dimensions that said living cells cannot pass through the second cross section, and (iii) a height between the first outer surface and the level of the second cross section such that either such an entire living cell or substantially such an entire living cell is containable within the aperture; and wherein the level of the second cross section is intermediate between the first and second outer surfaces of the carrier, or is coincident with the second outer surface of the carrier.

23. Apparatus according to claim 22, wherein said carrier is made of a metallic base with a superimposed coating of a material selected from the group consisting of an inorganic glass, Si, $SiO_2$, or a mixture of Si and $Si_2O_3$.

24. Apparatus according to claim 23, wherein the material of the metallic base is selected from the group consisting of copper, gold, nickel and silver.

25. Apparatus according to any of claims 10 to 24, wherein the positions on the carrier of the apertures are identifiable by means of a set of x and y coordinates.

26. Apparatus according to any of claims 22 to 25, which is further characterized in that it comprises apertures with side walls which extend between said first and second outer surfaces, the side walls including a first portion which converges inward towards the centre of the aperture and a second portion which is substantially perpendicular to said first and second outer surfaces.

27. Apparatus according to any of claims 10 to 26, wherein said carrier has been formed by a process which includes one of the following steps, namely:

creating apertures in a base by subjecting the base to ion bombardment through masks of different thicknesses; and

depositing metal on the back surface of a preformed grid comprising an ordered array of larger than cell-sized apertures, so as to progressively fill in these larger apertures until there is obtained an ordered array of apertures which are sized to hold individual cells.

28. A method according to any of claims 1 to 9, which is carried out in apparatus defined as in any of claims 22 to 27.

## Revendications

1. Procédé pour mettre en place des cellules vivantes individuelles à des emplacements identifiables, comprenant les étapes de :

(a) création d'un support sensiblement plan fait d'un matériau sélectionné dans le groupe constitué de : métal avec un revêtement inorganique superposé, plastique avec un revêtement inorganique superposé, métal non revêtu et plastique non revêtu, ledit support comportant une pluralité d'ouvertures qui sont disposées dans un réseau ordonné en deux dimensions et qui sont dimensionnées pour contenir les cellules individuelles ;

(b) application sur le support d'un fluide contenant les cellules vivantes ;

(c) application d'un champ électromagnétique aux cellules pour les amener dans les ouvertures ; et

(d) lavage du support pour retirer les cellules en excès et les débris.

2. Procédé selon la revendication 1, dans lequel, les cellules sont maintenues dans les ouvertures du support en exécutant une étape subséquente pour ajuster l'osmolarité du bain de solution entourant les cellules de manière à faire en sorte que les cellules gonflent.

3. Procédé selon soit la revendication 1 soit la revendication 2, dans lequel la force électromagnétique est produite par un champ électrique orienté perpendiculairement à la surface du support, ou bien par des champs électrique et magnétique croisés orientés parallèlement à la surface du support.

4. Procédé suivant l'une quelconque des revendications précédentes, dans lequel le bain de fluide entourant les cellules retenues dans les ouvertures du support est modifié, lors de l'application d'un champ magnétique variable dans le temps perpendiculairement à la surface du support pour faire tourner les cellules autour de leurs axes à l'intérieur des ouvertures.

5. Procédé selon l'une quelconque des revendications précédentes, qui comprend les étapes supplémentaires d'application d'un champ électrique variable dans le temps au travers du support et d'application d'un champ magnétique constant parallèlement à la surface du support, afin de déplacer les cellules ayant un rapport charge sur masse particulier hors de leurs ouvertures, ce par quoi de telles cellules sont séparées des cellules demeurant dans les ouvertures du support, et optionnellement lavage de surface du support pendant que lesdits champs électrique et magnétique sont appliqués.

6. Procédé selon l'une quelconque des revendications 1 à 4, dans lequel il est retiré au moins une cellule individuelle de son ouverture, par des étapes supplémentaires comprenant les étapes de chargement d'au moins une sonde et application à l'au moins une cellule du champ électrique local produit par l'au moins une sonde chargée pour déplacer l'au moins une cellule hors de son ouverture.

7. Procédé selon la revendication 6, dans lequel il est chargé un réseau de sondes et plusieurs cellules sont soumises aux champs électriques locaux produits par le réseau pour déplacer les cellules hors de leurs ouvertures respectives.

8. Procédé suivant l'une quelconque des revendications précédentes, dans lequel l'étape (d) est exécutée tout en appliquant une pression différentielle à travers le support pour maintenir les cellules dans les ouvertures.

9. Procédé suivant l'une quelconque des revendications précédentes, dans lequel l'étape (d) est exécutée en délivrant un fluide sur la surface supérieure du support au moyen d'un tube d'affluence et en le retirant au moyen d'un tube de drainage.

10. Appareil pour mettre en place des cellules individuelles à des emplacements identifiables comprenant :

(a) un support sensiblement plan fait d'un matériau sélectionné dans le groupe constitué de : métal avec un revêtement inorganique superposé, plastique avec un revêtement inorganique superposé, métal non revêtu et plastique non revêtu, ledit support comportant une pluralité d'ouvertures qui sont disposées dans un réseau ordonné en deux dimensions et qui sont dimensionnées pour contenir les cellules individuelles ;
(b) un moyen pour appliquer une force électromagnétique aux cellules pour les amener dans les ouvertures.

11. Appareil selon la revendication 10, dans lequel le moyen pour appliquer une force électromagnétique comprend un moyen pour produire au moins l'un des éléments suivants, à savoir :

un champ électrique orienté perpendiculairement à la surface du support ; et

des champs électrique et magnétique croisés parallèlement à la surface du support.

12. Appareil selon soit la revendication 10 soit la revendication 11, dans lequel ledit support est fait d'un matériau sélectionné dans le groupe constitué de : métal avec un revêtement inorganique superposé, plastique avec un revêtement inorganique superposé, ledit métal et ledit plastique étant conducteurs et ledit revêtement inorganique étant non-conducteur.

13. Appareil selon l'une quelconque des revendications 10 à 12, dans lequel le support est fait d'un métal et a une configuration qui localise les effets de l'électrolyse en des points distants des localisations des ouvertures.

14. Appareil selon la revendication 13, dans lequel le support comporte des saillies qui s'écartent de la partie du support qui contient les ouvertures.

15. Appareil selon l'une quelconque des revendications 10 à 14, qui comprend en outre des moyens pour délivrer au support le bain fluide des cellules et pour l'extraire du support.

16. Appareil selon la revendication 15, qui comprend également des moyens de production d'un champ magnétique variable dans le temps perpendiculaire à la surface du support pour faire en sorte que les cellules tournent autour de leurs axes à l'intérieur des ouvertures.

17. Appareil selon l'une quelconque des revendications 10 à 16, qui comprend en outre un moyen électromagnétique pour éjecter les cellules qui ont un rapport charge sur masse particulier d'une population de cellules capturées dans les ouvertures d'un support.

18. Appareil selon la revendication 17, dans lequel ledit moyen électromagnétique pour éjecter les cellules comprend un premier et un second moyens pour produire respectivement un champ électrique et un champ magnétique, ledit champ électrique étant variable dans le temps et étant dirigé au travers du support, ledit champ magnétique étant constant et étant dirigé parallèlement à la surface du support, ledit champ électrique étant adapté pour faire en sorte que les cellules qui ont un rapport charge sur masse particulier soient déplacées suffisamment loin de leurs ouvertures de manière à ce que ledit champ magnétique puisse déplacer les cellules dans un plan parallèle à la surface du support.

19. Appareil suivant soit la revendication 17, soit la revendication 18, qui comprend de plus un moyen pour laver la surface du support.

20. Appareil suivant l'une quelconque des revendications 10 à 19, qui comprend également un moyen pour retirer au moins une cellule contenue dans une ouverture du support comprenant au moins une sonde et un moyen pour charger au moins une sonde à une valeur suffisante pour attirer et déplacer l'au moins une cellule hors de la (les) ouverture(s).

21. Appareil selon la revendication 20, dans lequel ladite au moins une sonde comprend un réseau de sondes et un moyen pour charger des sondes sélectionnées dans le réseau à une valeur suffisante pour déplacer une pluralité de cellules hors de leurs ouvertures.

22. Appareil selon l'une quelconque des revendications 10 à 21, dans lequel ledit support définit une première et une seconde surfaces extérieures et comprend un réseau ordonné en deux dimensions d'ouvertures en travers de lui, les positions sur le support étant identifiables et les ouvertures étant dimensionnées pour contenir à l'intérieur des cellules vivantes individuelles dans lequel les ouvertures ont (i) une première section transversale à la première surface extérieure du support de dimensions telles que les cellules vivantes puissent passer à travers la première section transversale sans subir un dommage sensible, (ii) une seconde section transversale à un niveau espacé de la première surface extérieure de dimensions telles que les cellules vivantes ne puissent pas passer à travers la seconde section transversale, et (iii) une certaine hauteur entre la première surface extérieure et le niveau de la seconde section transversale de manière à ce que soit une telle cellule vivante entière soit sensiblement une telle cellule vivante entière puisse être contenue dans l'ouverture ; et dans lequel le niveau de la seconde section transversale est intermédiaire entre les première et seconde surfaces extérieures du support, ou coïncide avec la seconde surface extérieure du support.

23. Appareil selon la revendication 22, dans lequel ledit support est fait d'une base métallique avec un revêtement superposé d'un matériau choisi dans le groupe constitué d'un verre inor-

ganique, de Si, $SiO_2$ ou d'un mélange de Si et de $Si_2O_3$.

24. Appareil selon la revendication 23, dans lequel le matériau de la base métallique est sélectionné dans le groupe constitué du cuivre, de l'or, du nickel et de l'argent.

25. Appareil selon l'une quelconque des revendications 10 à 24, dans lequel les positions sur le support des ouvertures sont identifiables au moyen d'un ensemble de coordonnées x et y.

26. Appareil selon l'une quelconque des revendications 22 à 25, qui est en outre caractérisé en ce qu'il comprend des ouvertures avec des parois latérales qui s'étendent entre lesdites première et seconde surfaces extérieures, les parois latérales comprenant une première partie qui converge vers l'intérieur en direction du centre de l'ouverture et une seconde partie qui est sensiblement perpendiculaire auxdites première et seconde surfaces extérieures.

27. Appareil selon l'une des revendications 10 à 26, dans lequel ledit support a été formé par un processus qui comprend l'une des étapes suivantes, à savoir :

création d'ouvertures dans une base en soumettant la base à un bombardement ionique au travers de masques de différentes épaisseurs ; et

dépôt de métal sur la face arrière d'une grille préformée comprenant un réseau ordonné d'ouvertures dimensionnées plus grandes que les cellules, de manière à remplir progressivement ces ouvertures plus grandes jusqu'à ce qu'il soit obtenu un réseau ordonné d'ouvertures qui sont dimensionnées pour contenir les cellules individuelles.

28. Procédé selon l'une quelconque des revendications 1 à 9, qui est exécuté dans un appareil qui est défini comme dans l'une quelconque des revendications 22 à 27.

**Patentansprüche**

1. Verfahren zum Plazieren von einzelnen lebenden Zellen an identifizierbare Stellen, daß die Verfahrensschritte umfaßt:

(a) Schaffen eines im wesentlichen ebenen Trägers, der aus einem Material hergestellt ist, daß aus einer Gruppe ausgewählt wurde, die aus Metall mit einer überlagerten anorganischen Beschichtung, Kunststoff mit einer überlagerten anorganischen Beschichtung, unbeschichtetem Metall und unbeschichtetem Kunststoff besteht, wobei besagter Träger eine Vielzahl von Öffnungen aufweist, die in einer geordneten zweidimensionalen Anordnung angeordnet sind, und die so bemessen sind, um einzelne Zellen zu halten;

(b) Hinzufügen einer Flüssigkeit, die lebende Zellen enthält, zu dem Träger;

(c) Anlegen eines elektromagnetischen Feldes auf die Zellen, um die Zellen in die Öffnungen zu bewegen; und

(d) Waschen des Trägers, um überschüssige Zellen und Trümmer zu entfernen.

2. Verfahren gemäß Anspruch 1, worin die Zellen in den Öffnungen des Trägers gehalten werden, indem bei einem nachfolgenden Verfahrensschritt die Osmolarität der Badelösung, die die Zellen umgibt, so eingestellt wird, daß die Zellen anschwellen.

3. Verfahren gemäß Anspruch 1 oder Anspruch 2, worin die elektromagnetische Kraft durch ein elektrisches Feld hergestellt wird, das senkrecht zur Oberfläche des Trägers orientiert ist, oder durch sich kreuzende elektrische und magnetische Felder, die parallel zur Oberfläche des Trägers orientiert sind.

4. Verfahren gemäß einem der vorhergehenden Ansprüche, worin die Badeflüssigkeit, die die in den Öffnungen des Trägers gehaltenen Zellen umgibt, gewechselt wird, während ein zeitvariables magnetisches Feld senkrecht zu der Oberfläche des Trägers aufgetragen wird, um zu bewirken, daß sich die Zellen um ihre Achsen innerhalb der Öffnungen drehen.

5. Verfahren gemäß einem der vorhergehenden Ansprüche, daß weitere Verfahrensschritte umfaßt, nämlich Auftragen eines zeitvariablen elektrischen Feldes über den Träger und Auftragen eines konstanten magnetischen Feldes parallel zu der Oberfläche des Trägers, um Zellen aus ihren Öffnungen zu bewegen, die eine besondere spezifische Ladung haben, wodurch solche Zellen von Zellen separiert werden, die in den Öffnungen des Trägers verbleiben, und wahlweise Waschen der Oberfläche des Trägers, während besagte elektrische und magnetische Felder angelegt werden.

6. Verfahren gemäß einem der Ansprüche 1 bis 4, wobei durch weitere Verfahrensschritte mindestens eine einzelne Zelle aus ihrer Öffnung entfernt wird, die die Verfahrensschritte umfassen, nämlich Aufladen mindestens einer Sonde und Aussetzen mindestens einer Zelle dem lokalen elektrischen Feld, daß durch mindestens eine aufgeladene Sonde hergestellt wird, um mindestens eine Zelle aus ihrer Öffnung herauszubewegen.

7. Verfahren gemäß Anspruch 6, wobei eine Anordnung von Sonden aufgeladen wird und eine Vielzahl von Zellen den lokalen elektrischen Feldern ausgesetzt wird, die durch die Anordnung hergestellt werden, um die Zellen aus ihren jeweiligen Öffnungen herauszubewegen.

8. Verfahren gemäß einem der vorhergehenden Ansprüche, wobei der Verfahrensschritt (d) ausgeführt wird, während ein Differenzdruck über den Träger angelegt wird, um die Zellen in den Öffnungen zu halten.

9. Verfahren gemäß einem der vorhergehenden Ansprüche, wobei Verfahrensschritt (d) ausgeführt wird, indem Flüssigkeit zu der oberen Oberfläche des Trägers durch eine Zuflußleitung zugeführt und durch eine Abflußleitung entfernt wird.

10. Vorrichtung zur Plazierung einzelner Zellen an identifizierbaren Stellen, die umfaßt:

(a) einen im wesentlichen ebenen Träger, der aus einem Material hergestellt ist, das aus einer Gruppe ausgewählt wurde, die aus Metall mit einer übergelagerten anorganischen Beschichtung, Kunststoff mit einer übergelagerten anorganischen Beschichtung, unbeschichtetem Metall und unbeschichtetem Kunststoff besteht, wobei der Träger eine Vielzahl von Öffnungen aufweist, die in einer geordneten zweidimensionalen Anordnung angeordnet sind und die so bemessen sind, um einzelne Zellen zu halten;

(b) Vorrichtungen zum Auftragen einer elektromagnetischen Kraft auf die Zellen, um die Zellen in die Öffnungen zu bewegen.

11. Vorrichtung gemäß Anspruch 10, wobei die Vorrichtungen zum Auftragen einer elektromagnetischen Kraft Vorrichtungen einschließen, um mindestens eines von dem Folgenden herzustellen, nämlich:

ein elektrisches Feld, das senkrecht zu der Oberfläche des Trägers ausgerichtet ist; und sich kreuzende elektrische und magnetische Felder, die parallel zu der oberen Oberfläche des Trägers ausgerichtet sind.

12. Vorrichtung gemäß Anspruch 10 oder Anspruch 11, wobei besagter Träger aus einem Material hergestellt ist, das aus einer Gruppe ausgewählt wurde, die aus Metall mit einer überlagerten anorganischen Beschichtung, und Kunststoff mit einer überlagerten anorganischen Beschichtung besteht, wobei besagtes Metall und der Kunststoff Leiter sind und besagte anorganische Beschichtung ein Nichtleiter ist.

13. Vorrichtung gemäß einem der Ansprüche 10 bis 12, wobei der Träger aus Metall besteht und eine Struktur besitzt, die Elektrolyse-Wirkungen an Punkten lokalisiert, die sich im Abstand von den Stellen der Öffnungen befinden.

14. Vorrichtung gemäß Anspruch 13, wobei der Träger Vorsprünge mit einbezieht, die sich von dem Teil des Trägers weg erstrecken, der die Öffnungen mit einbezieht.

15. Vorrichtung gemäß einem der Ansprüche 10 bis 14, die zusätzliche Vorrichtungen einbezieht, um den Träger mit Zellbadeflüssigkeit zu versorgen und um diese von dem Träger abzuziehen.

16. Vorrichtung gemäß Anspruch 15, die ebenso Vorrichtungen zur Herstellung eines zeitvariablen Magnetfeldes senkrecht zur Oberfläche des Trägers mit einbezieht, um zu bewirken, daß sich die Zellen um ihre Achsen innerhalb der Öffnungen drehen.

17. Vorrichtung gemäß einem der Ansprüche 10 bis 16, die weiterhin elektromagnetische Vorrichtungen mit einbezieht, um Zellen herauszubefördern, die eine bestimmte spezifische Ladung unter der Population von Zellen aufweisen, die in den Öffnungen des Trägers gehalten werden.

18. Vorrichtung gemäß Anspruch 17, wobei besagte elektromagnetische Vorrichtungen zum Herausbefördern von Zellen erste und zweite Vorrichtungen umfassen, um entsprechend elektrische und magnetische Felder zu erzeugen, wobei besagtes elektrisches Feld zeitvariabel ist und über den Träger gerichtet ist, und wobei besagtes magnetisches Feld konstant ist und parallel zu der Oberfläche des Trägers gerichtet ist, und besagtes elektrisches Feld in der Lage ist, die Zellen, die eine bestimmte spezifische Ladung aufweisen, ausreichend weit aus ihren Öffnungen heraus zu bewegen, so daß besagtes magnetisches Feld die Zellen in einer zu der Oberfläche des Trägers parallelen Ebene bewegen kann.

19. Vorrichtung gemäß Anspruch 17 oder 18, die weiterhin Vorrichtungen zum Waschen der Oberfläche des Trägers umfaßt.

20. Vorrichtung gemäß einem der Ansprüche 10 bis 19, die weiterhin Vorrichtungen zum Entfernen mindestens einer Zelle mit einbezieht, die in einer Öffnung des Trägers enthalten ist, und mindestens eine Sonde und Vorrichtungen zum Aufladen mindestens der einen Sonde auf einen Wert umfaßt, der ausreichend ist, um mindestens die eine Zelle anzuziehen und aus der Öffnung (den Öffnungen) zu bewegen.

21. Vorrichtung gemäß Anspruch 20, wobei mindestens die eine besagte Sonde eine Anordnung von Sonden und Vorrichtungen zum Aufladen von mindestens ausgewählten Sonden in der Anordnung auf einen Wert umfaßt, der ausreichend ist, um eine Vielzahl von Zellen anzuziehen und aus ihren jeweiligen Öffnungen herauszubewegen.

22. Vorrichtung gemäß einem der Ansprüche 10 bis 21, wobei besagter Träger erste und zweite äußere Oberflächen definiert und eine geordnete zweidimensionale Anordnung von Öffnungen darauf umfaßt, wobei die Positionen der Öffnungen auf dem Träger identifizierbar sind und die Öffnungen so bemessen sind, um einzelne lebende Zellen aufzunehmen, wobei die Öffnungen (i) einen ersten Querschnitt an der ersten äußeren Oberfläche des Trägers mit einer solchen Größenordnung haben, daß lebende Zellen durch den ersten Querschnitt ohne wesentliche Beschädigung hindurch wandern können, und (ii) einen zweiten Querschnitt in einem Abstand von der ersten äußeren Oberfläche mit solchen Maßen haben, daß besagte lebende Zellen nicht durch den zweiten Querschnitt hindurch gelangen können, und (iii) eine Höhe zwischen der ersten äußeren Oberfläche und dem Niveau des zweiten Querschnittes haben, so daß entweder eine solche ganze lebende Zelle oder im wesentlichen eine solche lebende Zelle innerhalb der Öffnung aufnehmbar ist; und wobei das Niveau des zweiten Querschnittes in der Mitte zwischen der ersten und zweiten äußeren Oberfläche des Trägers liegt, oder mit der zweiten äußeren Oberfläche des Trägers übereinstimmend ist.

23. Vorrichtung gemäß Anspruch 22, wobei besagter Träger hergestellt ist aus einer metallischen Basis mit einer überlagerten Beschichtung aus einem Material, das aus der Gruppe ausgewählt wurde, die aus einem anorganischen Glas, Si, SiO$_2$, oder einer Mischung aus Si und

$Si_2O_3$ besteht.

24. Vorrichtung gemäß Anspruch 23, wobei das Material der metallischen Basis aus der Gruppe ausgewählt wurde, die aus Kupfer, Gold, Nickel und Silber besteht.

25. Vorrichtung gemäß einem der Ansprüche 10 bis 24, bei der die Positionen der Öffnungen auf dem Träger mittels eines Satzes von X- und Y-Koordinaten identifizierbar sind.

26. Vorrichtung gemäß einem der Ansprüche 22 bis 25, die weiterhin dadurch gekennzeichnet ist, daß sie Öffnungen mit Seitenwänden umfaßt, die sich zwischen besagten ersten und zweiten äußeren Oberflächen erstrecken, wobei die Seitenwände einen ersten Bereich, der nach innen in Richtung der Mitte der Öffnung zuläuft, und einen zweiten Bereich einbeziehen, der im wesentlichen senkrecht zu besagten ersten und zweiten äußeren Oberflächen liegt.

27. Vorrichtung gemäß einem der Ansprüche 10 bis 26, wobei besagter Träger durch ein Verfahren geformt wurde, daß einen der folgenden Verfahrensschritte umfaßt, nämlich:
Erzeugen von Öffnungen in einer Basis, indem die Basis einem Ionenbeschuß durch Masken verschiedener Dicke unterworfen wird; und Ablagern von Metall auf der hinteren Oberfläche eines vorgeformten Gitters, daß eine geordnete Anordnung von Öffnungen, die größer als die Zellen sind,umfaßt, um schrittweise in diese größeren Öffnungen zu füllen, bis eine geordnete Anordnung von Öffnungen geschaffen wurde, die so Bemessen sind, um einzelne Zellen zu halten.

28. Verfahren gemäß einem der Ansprüche 1 bis 9, daß in einer Vorrichtung durchgeführt wird, die in einem der Ansprüche 22 bis 27 definiert ist.

Fig.1A

Fig.1C

Fig.1B

Fig.1D

Fig.1E

Fig. 2

**Fig. 3**

Fig.4

Fig.5

Fig. 6

_Fig. 7_

Fig. 8

Fig.9

Fig.10

Fig.11

**Fig.12**

**Fig.13**

$B_{(t)}$

***Fig.14***

B

CELL MEMBRANE

$$F = Q\overline{V} \times \overline{B}$$

***Fig.15***

EP 0 162 907 B1

**Fig.16**